# EUROPEAN PATENT SPECIFICATION

(11) **EP 3 849 604 B1**
(45) Date of publication and mention of the grant of the patent: **01.05.2024**
(21) Application number: 19859381.6
(22) Date of filing: 10.09.2019
(51) Int. Cl.: A61K 39/395, A61K 31/505, C07K 16/26, C12N 15/44, C12N 15/45, A61K 38/16

(54) **GASTRIN-RELEASING PEPTIDE (GRP) INHIBITORS OR GASTRIN-RELEASING PEPTIDE RECEPTOR (GRPR) ANTAGONISTS FOR USE IN TREATING AN INFLUENZA VIRUS INFECTION**
INHIBITOREN DES GASTRIN-FREISETZENDEN PEPTIDS (GRP) ODER ANTAGONISTEN DES GASTRIN-FREISETZENDEN PEPTID-REZEPTORS (GRPR) ZUR VERWENDUNG BEI DER BEHANDLUNG EINER INFLUENZAVIRUSINFEKTION
INHIBITEURS DU PEPTIDE LIBÉRANT LA GASTRINE (GRP) OU D'ANTAGONISTES DU RÉCEPTEUR DU PEPTIDE LIBÉRANT LA GASTRINE (GRPR) POUR LE TRAITEMENT D'UNE INFECTION CAUSÉE PAR LE VIRUS DE LA GRIPPE

(30) Priority: 11.09.2018 US 201862729662 P
(43) Date of publication of application: 21.07.2021
(73) Proprietor: University of Maryland, Baltimore, Baltimore, MD 21201 (US); The United States of America, as represented by The Secretary, Department of Health and Human Services, Bethesda, MD 20892-7660 (US)
(72) Inventor: VOGEL, Stefanie N., Columbia, Maryland 21046-1305 (US); SHIREY, Kari Ann, Cockeysville, Maryland 21030 (US); CUTTITTA, Frank, Adamstown, Maryland 21710 (US)
(74) Representative: Dehns
(86) International application number: PCT/US2019/050312
(87) International publication number: WO 2020/055798

(56) References cited:
- WO-A1-2015/168682
- WO-A2-2006/063837
- US-A1- 2012 045 441
- US-A1- 2013 004 502
- Zhou Shutang ET AL: "EPITHELIAL AND MESENCHYMAL CELL BIOLOGY Radiation-Induced Lung Injury Is Mitigated by Blockade of Gastrin-Releasing Peptide", Am J Pathol, 4 April 2013 (2013-04-04), XP055907824, Retrieved from the Internet: URL:https://www.ncbi.nlm.nih.gov/pmc/artic les/PMC3620399/pdf/main.pdf [retrieved on 2022-03-31]
- SHIREY KARI ANN ET AL: "The TLR4 antagonist Eritoran protects mice from lethal influenza infection", NATURE, NATURE PUBLISHING GROUP UK, LONDON, vol. 497, no. 7450, 1 May 2013 (2013-05-01), pages 498-502, XP037115498, ISSN: 0028-0836, DOI: 10.1038/NATURE12118 [retrieved on 2013-05-01]
- MOODY et al.: "BW2258U89: a GRP receptor antagonist which inhibits small cell lung cancer growth", Life Sci, vol. 56, no. 7, 1995, pages 521-529, XP055085622, DOI: 10.1016/0024-3205(94)00481-7
- SHIREY et al.: "Novel role of gastrin releasing peptide-mediated signaling in the host response to influenza infection", Mucosal Immunol, vol. 12, no. 1, 16 October 2018 (2018-10-16), pages 223-231, XP036660124, DOI: 10.1038/s41385-018-0081-9

## Description

### TECHNICAL FIELD

The present invention generally relates to the field of medicine and in particular to the use of agents which are antagonists of gastrin-releasing peptide (GRP) or the gastrin-releasing peptide receptor (GRPR) for decreasing influenza-induced lethality and for attenuating the severity of lung inflammation associated with an influenza infection. The invention is defined by the appended claims.

### BACKGROUND OF INVENTION

Bombesin (BN) and gastrin-releasing peptide (GRP) are homologous amphibian/mammalian peptides, respectively, shown initially to up-regulate gastrin release and subsequent gastric acid secretion in the gut [1,2]. Tissue distribution of BN-like (BNL) or GRP immunoreactivities have been identified in the gastrointestinal tract, pancreas, adrenals, thyroid, brain, and lung [2]. The genes for GRP and its respective receptor (GRPR/BB2) have been cloned and their anatomical expression evaluated in healthy and disease states [1]. GRP is produced as an inactive "preform" that, after cleavage and amidation of the C-terminus, becomes the "mature," active form of GRP [1]. GRP/GRPR interaction has been demonstrated to mediate a variety of signal transduction pathways that include cAMP, MAPK, PI3K, and Akt, either directly or indirectly through the transactivation of other ligand/receptor systems [3].

Several different types of GRP inhibitors and/or GRPR antagonists have been developed as exploratory tools for mechanistic studies including compounds that either sequester the ligand or inhibit receptor binding. Examples include the neutralizing monoclonal antibody 2A11 (MoAb 2A11), a small-molecule inhibitor (NSC77427), and receptor-blocking agents like the water-soluble peptide BW2258U89 [4-7].

Over the past two decades, many studies have sought to identify the involvement of GRP in pulmonary disease. Inflammatory disorders like bronchopulmonary dysplasia (BPD), chronic obstructive pulmonary disease (COPD), chronic bronchitis, emphysema, and fibrosis have been shown to have a GRP regulatory component [2,8-10]. Distinct cells of the immune response are known to either produce or respond to GRP, underpinning macrophage activation and mast cell proliferation, migration, and degranulation [1,2,11]. Chronic cigarette smoking has been demonstrated to elevate GRP levels in the lung and also in the host urine [1,11]. For pulmonary neoplasms, GRP plays a major autocrine/paracrine growth modulatory role in small-cell lung cancer (SCLC) and non-small-cell lung cancer (NSCLC/adenocarcinoma) [1,12].

Together, these findings have led some to propose that GRP inhibitors or GRPR antagonists might attenuate the severity of lung inflammation. In a primate model of BPD, it was revealed by Sunday et al. [8] that treatment with MoAb 2A11, which only detects the mature (active, amidated) form of GRP [4], could abrogate the inflammation and arrest lung development characteristic of the disease [13]. Similarly, in a radiation-induced model of pneumonitis/fibrosis, lung injury was mitigated by therapeutic administration of the GRP inhibitor NSC77427 [14], that like MoAb 2011, only binds to the mature form of GRP [6].

It has been previously reported that mice expressing a targeted mutation in the gene that encodes Toll-like receptor 4 (TLR4) or therapeutic treatment of wild-type (WT) mice with TLR4 antagonists (small-molecule inhibitors or neutralizing antibody) effectively blocked viral-induced lethality in an experimental model of influenza infection [15-19]. Protection was associated with a blunting of the inflammatory response to infection, likely mediated by host-derived high-mobility group box-1 (HMGB1) activation of TLR4 [16,17]. TLR4 has also been shown to be involved in ozone-mediated and hyaluronan-mediated airway hyperresponsiveness, while TLR4-deficient animals are refractory [20]. More recently, ozone-induced airway hyperresponsiveness was shown to be inhibited by administration of MoAb 2A11, suggesting a role for GRP as well [21]. Interestingly, another GRPR peptide antagonist (RC-3095) was previously shown to inhibit TLR4 signaling and proposed as a possible therapeutic intervention strategy in sepsis [22]. Conversely, the low-affinity GRPR (NMBR/BB1) was shown to mediate macrophage Neu1 sialidase and matrix metalloproteinase-9 (MMP9) cross-talk inducing the transactivation of TLR-like receptors and cellular signaling [23].

Given the apparent link between TLR4 and GPR signaling, a better understanding of the role of GRP/GRPR in TLR4-mediated disease progression, such as that associated with influenza infection, may lead to novel treatments. The present invention is directed such important goals.

### BRIEF SUMMARY OF INVENTION

As discussed in detail herein, selected GRP inhibitors and GRPR antagonists were evaluated for their effectiveness in suppressing host lethality associated with the onset of viral pneumonia in a well-characterized mouse model of influenza. Influenza-induced lethality was blunted significantly by both GRP inhibitors and GRPR antagonists, and survival was accompanied by decreased numbers of GRP-producing pulmonary neuroendocrine cells (PNECs), improved lung histopathology, and suppressed pro-inflammatory cytokine gene expression. Together, these findings support the hypothesis that GRP contributes to influenza-induced disease, and the present invention is based on these important discoveries. In particular, inhibition of GRP or antagonizing GRPR during influenza infection represents a novel therapeutic approach to mitigating lung damage. The present invention generally encompasses GRP inhibitors and GRPR antagonists for the treatment of influenza based on these novel findings and observations, as set out in the appended claims.

In a first embodiment, the present invention is directed to a GRP inhibitor or a GRPR antagonist, or a combination thereof, for use in reducing a virally-induced inflammatory response in a subject infected with a virus, wherein the subject is infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89. In certain aspects of this use, the inflammatory response is pulmonary inflammation.

In a second embodiment, the present invention is directed to a GRP inhibitor or a GRPR antagonist, or a combination thereof, for use in reducing virally-induced lung damage in a subject infected with a virus, wherein the subject is infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89. In certain aspects of this use, the lung damage is one or more of degradation of airway parenchyma at bronchiole/alveoli foci due to infiltrating inflammatory cells and breakdown of pulmonary capillary integrity with generation of leaky vasculature.

In a third embodiment, the present invention is directed to a GRP inhibitor or a GRPR antagonist, or a combination thereof, for use in inhibiting virally-induced lethality of a subject infected with a virus and at risk for virally-induced lethality, wherein the subject is infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89.

In a fourth embodiment, the present invention is directed to a GRP inhibitor or a GRPR antagonist, or a combination thereof, for use in treating a viral infection in a subject infected with a virus, wherein the subject is infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89. In certain aspects of this use, treatment is a reduction in one or more of pulmonary inflammation, lung damage, decreased PNECs, GRP gene expression, GRP protein production, GRPR activation, GRPR signaling, inflammatory cytokine and/or chemokine production, and HMGB1 release in the subject in comparison to a subject infected with the virus but not receiving treatment.

In an fifth embodiment, the present invention is directed a GRP inhibitor or a GRPR antagonist, or a combination thereof, for use in treating viral pneumonia in a subject having viral pneumonia, wherein the subject is infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89. In certain aspects of this use, treatment is a reduction in one or more of pulmonary inflammation, lung damage, decreased PNECs, GRP gene expression, GRP protein production, GRPR activation, GRPR signaling, inflammatory cytokine and/or chemokine production, and HMGB1 release in the subject in comparison to a subject infected with the virus but not receiving treatment.

In uses of the invention, the influenza virus is an Influenza A virus, an Influenza B virus, an Influenza C virus or an Influenza D virus. Specific Influenza A viruses include, but are not limited to, the following serotypes or subtypes: H1N1 (including the California pH1N1 strain), H1N2, H2N2, H2N3, H3N1, H3N2 (including the Wuhan H3N2 and Victoria H3N2 strains), H3N8, H5N1, H5N2, H5N3, H5N6, H5N8, H5N9, H6N1, H6N2, H7N1, H7N2, H7N3, H7N4, H7N7, H7N9, H9N2, and H10N7. Specific Influenza B viruses include, but are not limited to, the following serotypes or subtypes: Victoria and Yamagata.

The foregoing has outlined rather broadly the features and technical advantages of the present invention in order that the detailed description of the invention that follows may be better understood. Additional features and advantages of the invention will be described herein, which form the subject of the claims of the invention. It should be appreciated by those skilled in the art that any conception and specific embodiment disclosed herein may be readily utilized as a basis for modifying or designing other structures for carrying out the same purposes of the present invention. The novel features which are believed to be characteristic of the invention, both as to its organization and operation, together with further objects and advantages will be better understood from the following description when considered in connection with the accompanying figures. It is to be expressly understood, however, that any description, figure, example, etc. is provided for the purpose of illustration and description only. The scope of the invention is set forth in the appended claims.

### BRIEF DESCRIPTION OF DRAWINGS

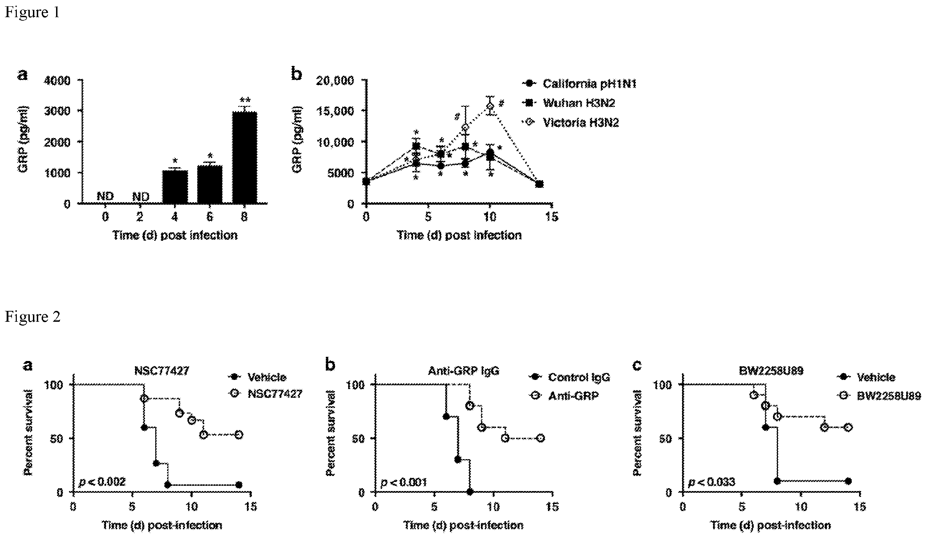

**Figure 1****.** Influenza infection of mice and cotton rats induces GRP in lungs and serum. **a** WT C57BL/6J mice were infected with mouse-adapted influenza strain PR8 (LD₉₀; -7500 TCID₅₀). Mice were euthanized on days 0, 2, 4, 6, and 8 post-infection (3-5 mice/group; **p* < 0.001; ***p* < 0.0002). Lungs were homogenized and processed for GRP levels by EIA according to the manufacturer's protocol. **b** Cotton rats were infected i.n. with 1 × 10⁶ TCID₅₀ of either California pH1N1, and Wuhan H3N2, or 1 × 10⁵ TCID₅₀ of Victoria H3N2, and serum GRP levels were analyzed at the indicated days p.i. **p* < 0.05 and *p* < 0.01 for comparison between day 0 GRP (*N* = 23) vs. each time p.i. for each of the different influenza A strains.
**Figure 2****.** Blocking GRP and GRPR enhances survival after influenza infection. **a** WT C57BL/6J mice were infected with mouse-adapted influenza strain PR8 (LD₉₀; -7500 TCID₅₀). Mice received vehicle (saline + 0.096% DMSO) or GRP inhibitor (NSC77427; 20 µM, 100 µl i.v./mouse) daily from day 2 to day 6 post-infection. Survival was monitored for 14 days. Data shown are combined results of three assays (5 mice/treatment group/experiment). **b** WT C57BL/6J mice were infected as described in **a.** Mice received either control IgG or a highly specific anti-GRP IgG (100 µg; 100 µl i.v./mouse) on days 2 and day 4 post-PR8 infection. Survival was monitored as in **a.** Data shown are combined results of two assays (5 mice/treatment group/experiment). **c** WT C57BL/6J mice were infected as described in **a.** Mice received vehicle (saline) or GRPR antagonist (BW2258U89; 20 µM, 100 µl i.v./mouse). Survival was monitored as in **a.** Data shown are combined results of two assays (5 mice/treatment group/experiment).

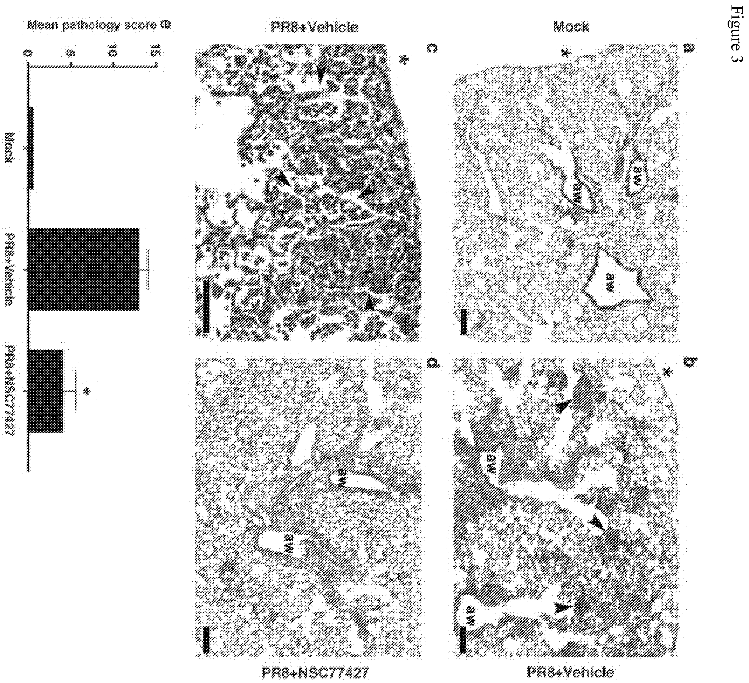

**Figure 3****.** GRP inhibitor, NSC77427, reduces lung pathology after influenza PR8 challenge. WT C57BL/6J mice (5 mice/treatment group) were infected i.n. with mouse-adapted influenza strain PR8 (LD₉₀; -7500 TCID₅₀). Mice received vehicle (saline + 0.0096% DMSO) or GRP inhibitor (NSC77427; 20 µM/mouse; i.v.) daily from days 2 to 6 post-infection. On day 7 post-infection, mice were euthanized and lungs were extracted, fixed, and stained for histopathology. Photomicrographs of representative sections were taken at × 10 (**a, b-d**), and at ×40 (c). All scale bars are 100 µm. N = 4 mice/group. Asterisk indicates the pleural surface. **e** Quantitation of lung injury is based on the scoring system detailed in the Materials section. Data shown are mean ± SD. **p* = 0.002.

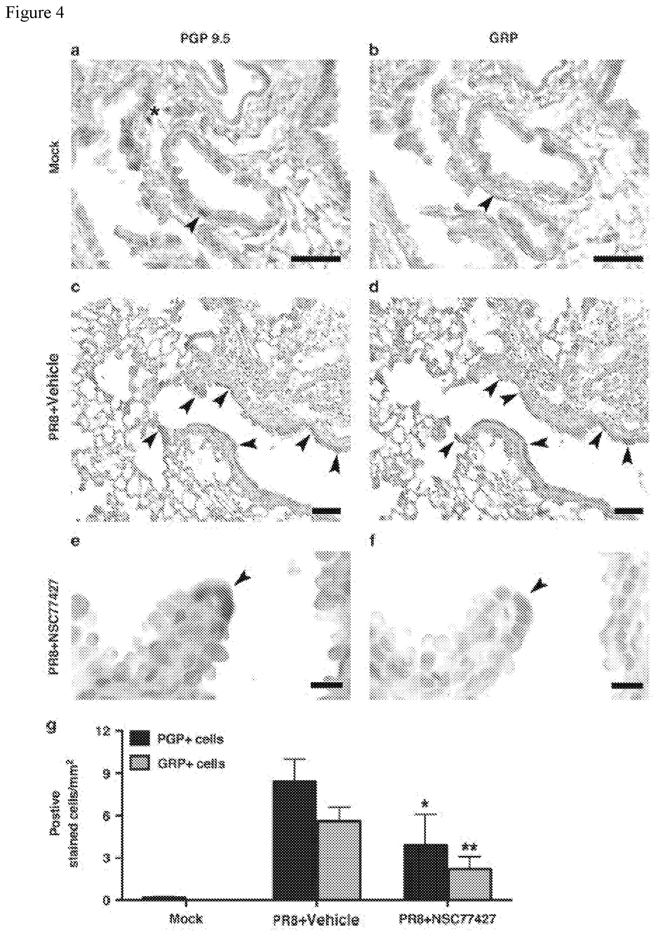

**Figure 4****.** IHC staining for pulmonary neuroendocrine cells (PNECs), PGP 9.5, and GRP in influenza-infected mice. Lung sections from Figure 3 were stained in **a** mock-infected, **c** PR8 + vehicle, and **e** PR8 + NSC77427 for neural/neuroendocrine-specific PGP 9.5, or in **b** mock-infected, **d** PR8 + vehicle, and **f** PR8 + NSC77427 for GRP, a neuroendocrine granule marker. Scale bars shown are 50 µm for **a-d**, and 10 µm for **e** and **f.** Arrows are positioned in each panel within a treatment group to indicate groups of positive cells, except in **b,** where the arrow indicates GRP- cells adjacent to the PGP 9.5+ cells shown in **a. g** Graphic image analysis of PGP 9.5+ and GRP+ cells after IHC staining. Slides were blinded by an observer with no knowledge of experimental groups. PCP9.5+ and GRP+ cells were counted throughout each complete lung lobe crosssection for each animal, with validation by ×40 micrographs of each slide. Total tissue area was determined by using ImageI thresholding analysis (methyl green counterstain) because PNECs are mainly observed in alveolar ducts and small bronchioles. **p* = 0.009; ***p* = 0.001

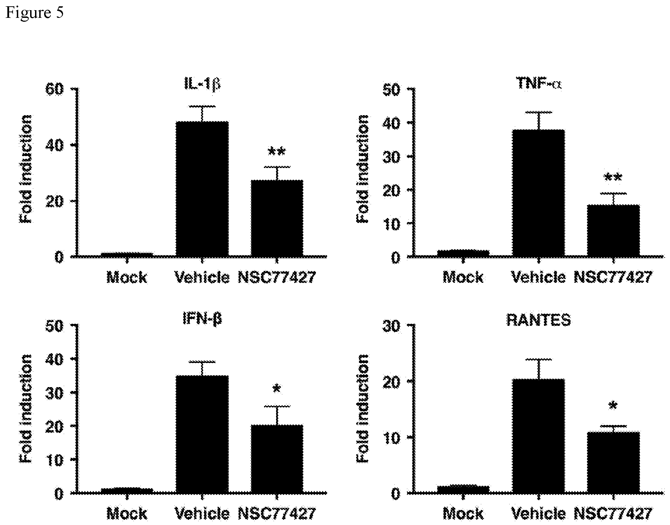

**Figure 5****.** Blocking GRP blunts influenza-induced cytokine induction *in vivo.* WT C57BL/6J mice (5 mice/treatment group) were infected i.n. with mouse-adapted influenza strain PR8 (LD₉₀; -7500 TCID₅₀). Mice received vehicle (saline + 0.0096% DMSO) or GRP inhibitor (NSC77427; 20 µM/mouse; i.v.) daily from day 2 to day 6 post infection. On day 7 post-infection, lungs were harvested and total RNA was extracted to measure gene expression by qRT-PCR. ***p* < 0.01; **p* < 0.05.

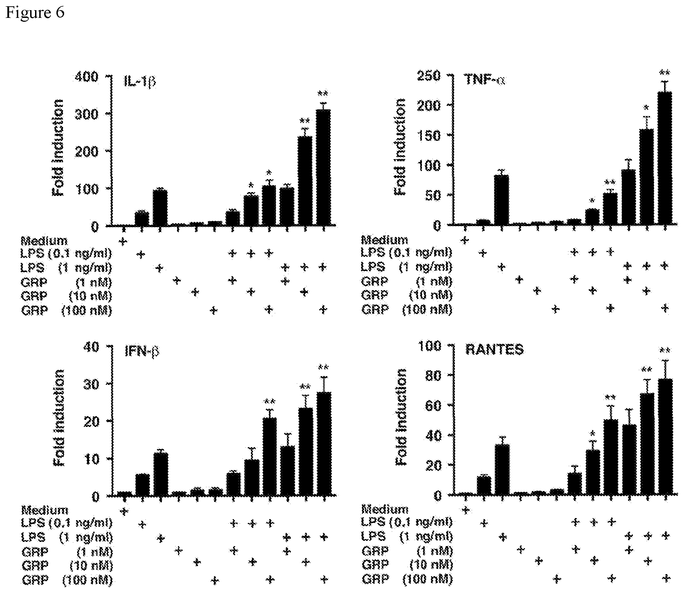

**Figure 6****.** GRP enhances LPS signaling. WT C57BL/6J peritoneal macrophages were treated with medium only or LPS (0.1 or 1 ng/ml) in the absence or presence of increasing GRP doses (1, 10, or 100 nM, respectively) for 2 h and mRNA expression measured. Data represent the means ± SEM from two separate experiments (**p* < 0.01; ***p* < 0.001 compared to induction of gene expression with LPS treatment alone).

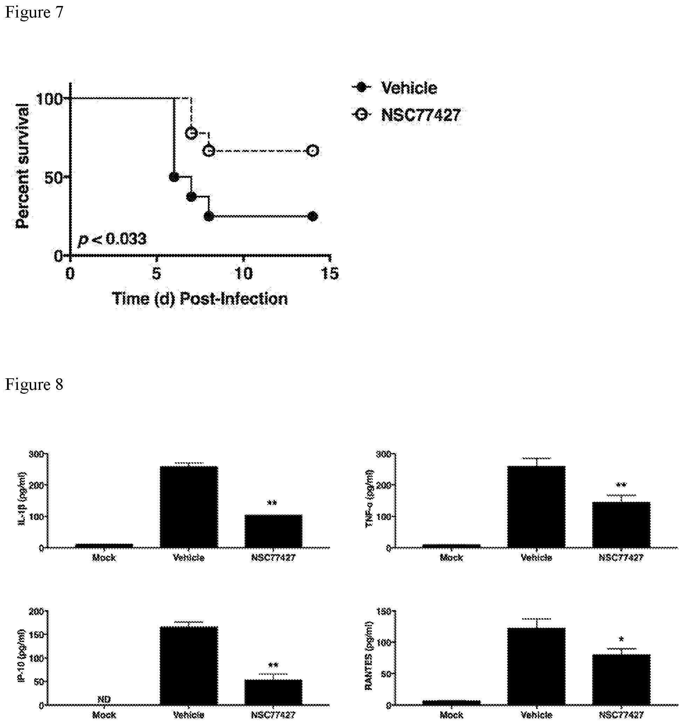

**Figure 7****.** Inhibition of GRP enhances survival of mice after A/California/07/2009 H1N1 infection. WT C57BL/6J mice were infected (i.n.) with ∼10⁷ TCID₅₀ A/California/07/2009 influenza strain. Mice received vehicle (saline + 0.096% DMSO) or GRP antagonist (NSC77427; 20 µM, 100 µl i.v./mouse) once daily from day 2 to day 6 post-infection. Survival was monitored for 14 days (8 mice/treatment group).
**Figure 8****.** Inhibition of GRP blunts influenza-induced cytokine protein levels *in vivo.* WT C57BL/6J mice (5 mice/treatment group) were infected i.n. with mouse-adapted influenza strain PR8 (LD₉₀; -7500 TCID₅₀). Mice received vehicle (saline + 0.0096% DMSO) or GRP antagonist (NSC77427; 20 µM/mouse; i.v.) once daily from day 2 to day 6 post-infection. On day 7 post-infection, lungs were harvested and cytokine protein levels measured in lung homogenates. ***p* < 0.01. **p* < 0.05.

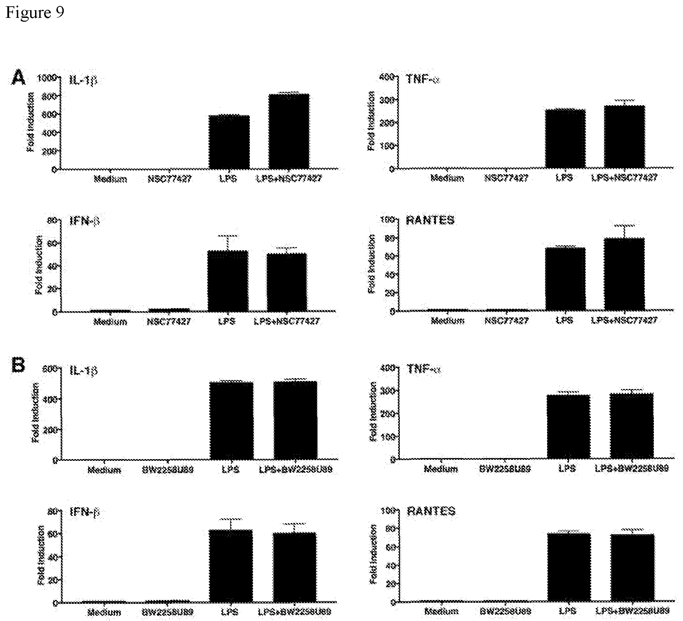

**Figure 9****.** Neither GRP inhibition nor GRPR antagonism inhibit LPS signaling *in vitro.* A WT C57BL/6J peritoneal macrophages were pre-treated with NSC77427 (0.5 µM) for 1 h. LPS (10 ng/ml) was added and cells incubated for an additional 2 h and gene expression analyzed. **B** WT C57BL/6J peritoneal macrophages were pre-treated with BW2258U89 (1 µM) for 1 h. LPS (10 ng/ml) was added and cells incubated for an additional 2 h and gene expression analyzed. Data shown are mean +/- SEM from two separate experiments.

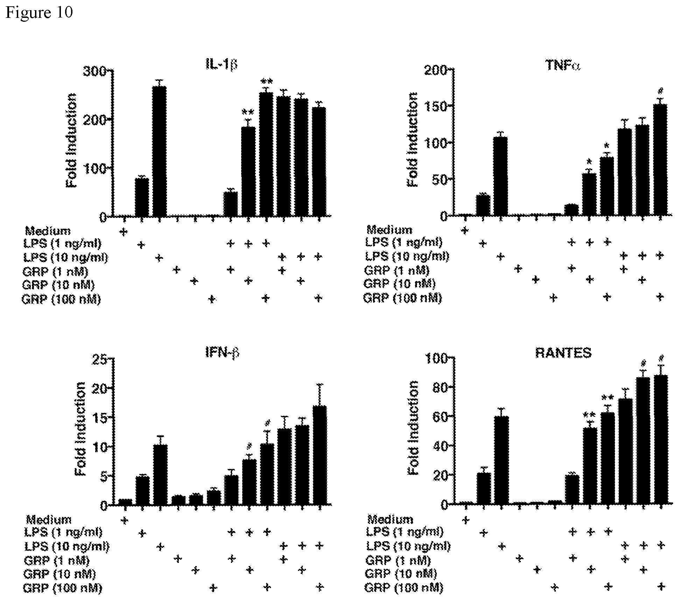

**Figure 10****.** GRP enhances both MyD88- and TRIF-dependent signaling induced by suboptimal concentrations of LPS. WT C57BL/6J peritoneal macrophages were treated with LPS at 1 ng/ml or 10 ng/ml in the absence or presence of increasing GRP doses (1 nM, 10 nM, or 100 nM) for 2 h and mRNA expression measured. (# *p* < 0.05; * *p* < 0.01; ** *p* < 0.001 compared to induction of gene expression with LPS treatment alone).

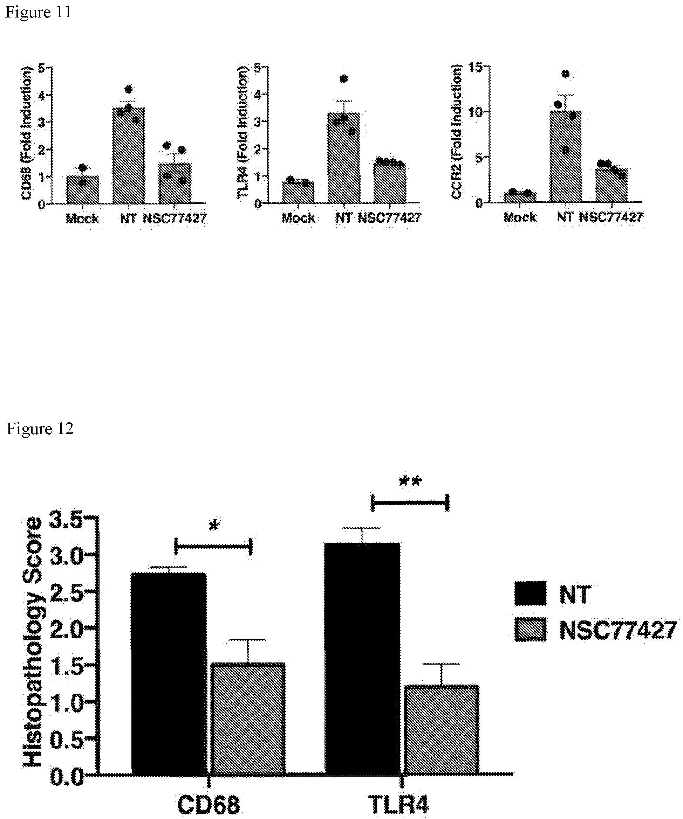

**Figure 11****.** Illustrated are mRNA levels for CD68 (a macrophage marker), TLR4, and CCR2 (a marker of infiltrating monocytes) in RNA prepared from lungs of mice that were untreated (mock), infected but not treated (NT), or infected and treated with the GRP inhibitor NSC77427. Mice were mock infected or infected with influenza A/PR/8/34 (PR8) and then treated with saline (NT) or NSC77427 once daily from day 2 to day 6 post-infection. Each point represents an individual mouse.
**Figure 12****.** Immunohistochemistry (IHC) for the detection of CD68 and TLR4 was carried out on lung sections from the same mice as shown in Figure 11. The numbers on the y-axis represent the mean ±SEM of the number of cells per 20X field that were stained with anti-CD69 or anti-TLR4 antibody. **p* = 0.0117; ***p* = 0.0069.

### DETAILED DESCRIPTION OF THE INVENTION

### I. Definitions

As used herein, "a" or "an" may mean one or more. As used herein when used in conjunction with the word "comprising," the words "a" or "an" may mean one or more than one. As used herein "another" may mean at least a second or more. Furthermore, unless otherwise required by context, singular terms include pluralities and plural terms include the singular.

As used herein, "about" refers to a numeric value, including, for example, whole numbers, fractions, and percentages, whether or not explicitly indicated. The term "about" generally refers to a range of numerical values (e.g., +/- 5-10% of the recited value) that one of ordinary skill in the art would consider equivalent to the recited value (e.g., having the same function or result). In some instances, the term "about" may include numerical values that are rounded to the nearest significant figure.

### II. The Present Invention

Over 40 years ago, the lung was characterized as a new endocrine organ that produced classic peptide hormones in distinct cells of the bronchiole columnar epithelium, namely the pulmonary neuroendocrine cell (PNEC) [27]. Subsequent to this discovery, immunoreactive amphibian BNL activity was identified in human fetal PNEC and later determined to be mammalian homolog, gastrin-releasing peptide (GRP) [11,28]. Like most peptide hormones, GRP is initially produced in the cell as its inactive pre-proform, enzymatically processed in the Golgi cisterna and secretory granules, and then released to the external milieu as mature bioactive methionine-amide GRP [29]. The potential for endocrine regulation of the immune response was first implicated in 1985 and has since been shown to be a dynamic bidirectional process where immune cells can make and respond to peptide hormones [30-32]. BNL/GRP has been shown to not only stimulate fetal lung growth/maturation, but also to contribute to a variety of pulmonary inflammatory diseases and malignancies including BPD, COPD, emphysema, fibrosis, SCLC, and NSCLCs [1,11,12]. However, relatively little attention has been given to the potential role of GRP in lung inflammation of infectious etiology.

Influenza infection poses a global threat. Despite the availability of annual vaccines, the need to predict the major strains to be included in each upcoming year's vaccine, as well as the appearance of strains to which humans have had no prior exposure, has led to an ongoing effort by many to develop a "universal influenza vaccine" [33,34]. In addition, influenza viruses have mutated to become resistant to many of the drugs in the current arsenal of antiviral agents [35,36]. Moreover, antiviral therapies must be administered relatively early in infection to be effective [37]. Therefore, the present invention is focused on alternative therapeutic strategies that act by modulating the host's innate immune response to influenza infection. To date, the inventors have shown that antagonizing TLR4 signaling therapeutically is efficacious in both murine and cotton rat models of influenza. The TLR4 agonist activity induced by influenza infection is attributable, in large part, to the action of a host-derived "danger-associated molecular pattern," HMGB1. HMGB1, a chromatin-associated molecule, is often released from dying cells and has been shown to be a TLR4 agonist [38]. Demonstrated herein for the first time, GRP plays an active role in regulating host lethality and lung pathology in a mouse model of influenza.

As discussed in detail in the Examples below, the cellular mechanism underlying GRP induction during influenza infection is revealed. The data presented herein strongly supports the observation that both the number of PGP 9.5+ PNECs and the number of GRP+ PNECs was increased upon viral infection, indicating a direct effect on the differentiation of these specialized epithelial airway cells, which has been reported to result from the interplay of increased canonical Wnt signaling and diminished Notch signaling [39,40]. Together, these observations suggest that GRP may provide a "feed-forward" signal that promotes PNEC differentiation and/or its own synthesis, consistent with observations in fetal baboon lung [41]. In addition, influenza infection has been shown to activate Wnt signaling, perhaps facilitating the production of GRP [42]. In this regard, Shapira et al. [42] found that treatment of human bronchial epithelial cells with recombinant WNT protein increased cellular production of IFN-β in response to influenza infection.

Oxidative stress or acute lung injury can mediate PNEC hyperplasia accompanied by enhanced GRP expression in these neuroendocrine cells followed by release of this hormone into the surrounding airway parenchyma [11,43]. GRPR, in turn, is known to mediate neutrophil chemotaxis and lung macrophages/PMN infiltrates that can produce the regional release of MMP2/MMP9. These contribute to the breakdown of basement membrane integrity and the altering of bronchiole/alveolar architecture, diminution of pulmonary function, and the onset of disease lethality in COPD and lung fibrosis [26,44-46]. Similar pathological events are thought to occur in the animal model of influenza present herein, and targeting GRP with appropriate inhibitors/antagonists disrupts disease progression.

As shown herein, therapeutic administration of the GRP inhibitor, NSC77427, inhibits cytokine production in whole lung homogenates from PR8-infected mice. Thus, the inflammatory response to PR8 infection appears to be mediated, in part, by the action of GRP on both PNEC differentiation and cytokine gene expression.

Taken together, the data presented herein suggests that during PR8 infection other cell types, for example, PNECs, are likely to be the major source of GRP, and that administration of the GRP inhibitor blocks its ability to synergize with TLR4 signaling to elicit a more potent cytokine storm, leading to lung pathology and death. The findings presented herein suggest that GRP is a neuroendocrine peptide that acts as a novel contributor to the inflammatory response to influenza infection. Thus, inhibition of GRP or antagonizing the GRPR during influenza infection represents a novel therapeutic approach to mitigating lung damage. The present invention is concerned with a GRP inhibitor or a GRPR antagonist, as defined herein, or a combination thereof, for use based on these novel findings and observations.

### Reducing Virally-Induced Inflammatory Responses

The present invention is directed, in part, to a GRP inhibitor or a GRPR antagonist, or a combination thereof, for use in reducing a virally-induced inflammatory response in a subject infected with a virus, comprising administering a therapeutically effective amount of a GRP inhibitor or a GRPR antagonist, or a combination thereof, to a subject infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89. In certain aspects of this use, the inflammatory response is pulmonary inflammation.

### Reducing Virally-induced Lung Damage

The present invention is directed, in part, to a GRP inhibitor or a GRPR antagonist, or a combination thereof, for use in reducing virally-induced lung damage in a subject infected with a virus, comprising administering a therapeutically effective amount of a GRP inhibitor or a GRPR antagonist, or a combination thereof, to a subject infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89. In certain aspects of this use, the lung damage is one or more of degradation of airway parenchyma at bronchiole/alveoli foci due to infiltrating inflammatory cells and breakdown of pulmonary capillary integrity with generation of leaky vasculature.

### Inhibiting Virally-Induced Lethality

The present invention is directed, in part, to a GRP inhibitor or a GRPR antagonist, or a combination thereof, for use in inhibiting virally-induced lethality of a subject infected with a virus and at risk for virally-induced lethality, comprising administering a therapeutically effective amount of a GRP inhibitor or a GRPR antagonist, or a combination thereof, to a subject infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89.

Virally-induced lethality is death of the subject that is the direct or indirect result of a viral infection in the subject by an influenza virus as defined herein.

### Treating Viral Infection

The present invention is directed, in part, to a GRP inhibitor or a GRPR antagonist, or a combination thereof, for use in treating a viral infection in a subject infected with a virus, comprising administering a therapeutically effective amount of a GRP inhibitor or a GRPR antagonist, or a combination thereof, to a subject infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89. In certain aspects of this use, treatment is a reduction in one or more of pulmonary inflammation, lung damage, decreased PNECs, GRP gene expression, GRP protein production, GRPR activation, GRPR signaling, inflammatory cytokine and/or chemokine production, and HMGB1 release in the subject in comparison to a subject infected with the virus but not receiving treatment.

### Treating Viral Pneumonia

The present invention is directed, in part, to a GRP inhibitor or a GRPR antagonist, or a combination thereof, for use in treating viral pneumonia in a subject having viral pneumonia, comprising administering a therapeutically effective amount of a GRP inhibitor or a GRPR antagonist, or a combination thereof, to a subject infected with an influenza virus, wherein the influenza virus is an Influenza A virus, an Influenza B virus, an Influenza C virus or an Influenza D virus, wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and wherein the GRPR antagonist is the water-soluble peptide BW2258U89. In certain aspects of this use, treatment is a reduction in one or more of pulmonary inflammation, lung damage, decreased PNECs, GRP gene expression, GRP protein production, GRPR activation, GRPR signaling, inflammatory cytokine and/or chemokine production, and HMGB1 release in the subject in comparison to a subject infected with the virus but not receiving treatment.

### GRP inhibitors and GRPR antagonists

The GRP inhibitors that are used in the invention are those as set out in the appended claims and may have binding specificity for GRP that reduces the activity of the protein by at least 10, 15, 20, 25, 30, 35, 40, 45, 50, 55, 60, 65, 70, 75, 80, 85, 90 or 95%, or more. Relevant GRP activity includes binding and/or activation of GRPR. The GRP inhibitors used in the invention are the mouse anti-GRP neutralizing monoclonal antibody 2A11 (MoAb 2A11, an antibody with binding specificity for GPR), the small-molecule inhibitor NSC77427 (2-[(2-amino-6-chloropyrimidin-4-yl)amino]ethanol; PubChem CID: 235898), NSC54671 (3-[2-(5-oxidooxadiazol-3-ium-3-yl)ethyl]oxadiazol-3-ium-5-olate; PubChem CID: 574434), NSC112200 (2,4-dibromo-3,6-dimethylbenzene-1, 4-diol; PubChem CID: 270077), and 2,5-dibromo-3,6-dimethylcyclohexa-2,5-diene-1,4-dione). These inhibitors of GRP are listed in [51] and US Patent Publication 20120232107.

The GRPR antagonists that may be used in the invention are those as set out in the appended claims and may have binding specificity for GRPR that reduces at least one activity of the receptor by at least 10, 15, 20, 25, 30, 35, 40, 45, 50, 55, 60, 65, 70, 75, 80, 85, 90 or 95%, or more. The invention uses the water-soluble peptide BW2258U89.

Antibodies having binding specificity for GPR are monoclonal or GRP-binding fragments thereof. The fragments include, but are not limited to, Fab fragments, F(ab')₂ fragments, single chain Fv (scFv) antibodies, and fragments produced by a Fab expression library, as well as bi-specific antibody and triple-specific antibodies. The humanized antibodies include fully human antibodies. The antibodies may be produced in any species of animal, though preferably from a mammal such as a human, simian, mouse, rat, rabbit, guinea pig, horse, cow, sheep, goat, pig, dog or cat. For example, the antibodies can be human or humanized, or any binding agent preparation suitable for administration to a human.

### Viruses

In each aspect and embodiment of the invention, the virus is a virus that causes a respiratory infection, that is, an infection of the respiratory system of the subject, including, but not limited to a viral infection of the lungs, including one or more of the bronchus, bronchi, bronchioles, alveolar ducts, alveolus, and alveoli; nasal cavity; pharynx; larynx; trachea; parietal pleura; and visceral pleura.

In uses of the invention, the influenza virus is an Influenza A virus, an Influenza B virus, an Influenza C virus or an Influenza D virus. Specific Influenza A viruses include, but are not limited to, the following serotypes or subtypes: H1N1 (including the California pH1N1 strain), H1N2, H2N2, H2N3, H3N1, H3N2 (including the Wuhan H3N2 and Victoria H3N2 strains), H3N8, H5N1, H5N2, H5N3, H5N6, H5N8, H5N9, H6N1, H6N2, H7N1, H7N2, H7N3, H7N4, H7N7, H7N9, H9N2, and H10N7. Specific Influenza B viruses include, but are not limited to, the following serotypes or subtypes: Victoria and Yamagata.

The uses of the invention may be practiced by administering one or more GRP inhibitor to the subject, administering one or more GRPR antagonist to the subject, or administering a combination of one or more GRP inhibitor and one or more GRPR antagonist to the subject, wherein the GRP inhibitor and/or GRPR antagonist are as described in the appended claims.

While the GRP inhibitors and/or GRPR antagonists may be administered directly to a subject, the uses of the present invention are preferably based on the administration of a pharmaceutical formulation comprising the one or more of the inhibitors and/or antagonists and a pharmaceutically acceptable carrier or diluent. Thus, pharmaceutical formulations comprising one or more of the inhibitors and/or antagonists defined herein and a pharmaceutically acceptable carrier or diluent may be used.

Pharmaceutically acceptable carriers and diluents are commonly known and will vary depending on the particular inhibitor or antagonist being administered and the mode of administration. Examples of generally used carriers and diluents include, without limitation: saline, buffered saline, dextrose, water-for-injection, glycerol, ethanol, and combinations thereof, stabilizing agents, solubilizing agents and surfactants, buffers and preservatives, tonicity agents, bulking agents, and lubricating agents. The formulations comprising inhibitors or antagonists will typically have been prepared and cultured in the absence of any non-human components, such as animal serum (e.g., bovine serum albumin).

Pharmaceutical formulations comprising one or more inhibitors and antagonists may be administered to a subject using modes and techniques known to the skilled artisan. Characteristic of pulmonary infections may make it more amenable to administer the formulations, inhibitors and antagonists using means that allow targeting to the respiratory system. Suitable modes of delivery include, but are not limited to, aerosol administration via the mouth or nose. Other suitable modes include, without limitation, intradermal, subcutaneous (s.c., s.q., sub-Q, Hypo), intramuscular (i.m.), intraperitoneal (i.p.), intra-arterial, intramedulary, intracardiac, intra-articular (joint), intrasynovial (joint fluid area), intracranial, intraspinal, and intrathecal (spinal fluids).

Depending on the means of administration, the dosage may be administered all at once, such as with an oral formulation in a capsule or liquid, or slowly over a period of time, such as with an intramuscular or intravenous administration.

The amount of inhibitors and antagonists, alone or in a pharmaceutical formulation, administered to a subject is an amount effective for practicing the uses of the invention. In the various embodiments and aspects of the invention, the therapeutically effective amount of a GRP inhibitor or a GRPR antagonist, or a combination thereof, is one that brings about the desired outcome of the use practiced, namely the reducing, inhibiting or treating as recited in the various embodiments and aspects of the invention. Thus, therapeutically effective amounts are administered to subjects when the uses of the present invention are practiced.

In general, between about 1 ug/kg and about 1000 mg/kg of the inhibitor or antagonist per body weight of the subject is administered. Suitable ranges also include between about 50 ug/kg and about 500 mg/kg, and between about 10 ug/kg and about 100 mg/kg. However, the amount of inhibitor or antagonist administered to a subject will vary between wide limits, depending upon the location, source, extent and severity of the infection, the age and condition of the subject to be treated, etc. A physician will ultimately determine appropriate dosages to be used.

Administration frequencies of the inhibitors, antagonists, and pharmaceutical formulations comprising the same will vary depending on factors that include the location of the viral infection, the particulars of the infection to be treated or prevented, and the mode of administration. Each inhibitor, antagonist, or formulation may be independently administered 4, 3, 2 or once daily, every other day, every third day, every fourth day, every fifth day, every sixth day, once weekly, every eight days, every nine days, every ten days, bi-weekly, monthly and bimonthly.

The duration of the use practiced will be based on location and severity of the infection, and will be best determined by the attending physician. However, continuation of treatment is contemplated to last for a number of days, weeks, or months.

As used herein, the terms "reduce", "reducing", and "reduction" have their ordinary and customary meanings, and include one or more of diminishing or decreasing: the degree of a virally-induced inflammatory response in a subject; and the amount of virally-induced lung damage in a subject, in comparison to a subject in which the uses of the present invention have not been practiced. Reducing means diminishing or decreasing by about 50% to about 100% in comparison to a subject in which the uses of the present invention have not been practiced. Preferably, the diminishing or decreasing is about 100%, 99%, 98%, 97%, 96%, 95%, 90%, 80%, 70%, 60%, or 50% in comparison to a subject in which the uses of the present invention have not been practiced.

As used herein, the terms "inhibit", "inhibiting", and "inhibition" have their ordinary and customary meanings, and include one or more of averting or obstructing virally-induced lethality in a subject, in comparison to a subject in which the uses of the present invention have not been practiced. Inhibiting means averting or obstructing by about 50% to about 100% in comparison to a subject in which the uses of the present invention have not been practiced. Preferably, the averting or obstructing is about 100%, 99%, 98%, 97%, 96%, 95%, 90%, 80%, 70%, 60%, or 50% in comparison to a subject in which the uses of the present invention have not been practiced.

As used herein, the terms "treat", "treating", and "treatment" have their ordinary and customary meanings, and include one or more of: blocking, ameliorating or decreasing in severity and/or frequency a symptom of a viral infection in a subject, such as viral pneumonia. Treatment means blocking, ameliorating or decreasing by about 50% to about 100% in comparison to a subject in which the uses of the present invention have not been practiced. Preferably, the blocking, ameliorating or decreasing is about 100%, 99%, 98%, 97%, 96%, 95%, 90%, 80%, 70%, 60%, or 50% in comparison to a subject in which the uses of the present invention have not been practiced.

In each embodiment and aspect of the invention, the subject is a human, a non-human primate, pig, bird, horse, cow, goat, sheep, a companion animal, such as a dog, cat or rodent, or other mammal.

A kit may be prepared comprising one or more containers filled with one or more of the inhibitors and/or antagonists and/or pharmaceutical formulations comprising inhibitors and/or antagonists. The kit may include instructions for use. Associated with the kit may further be a notice in the form prescribed by a governmental agency regulating the manufacture, use or sale of pharmaceuticals or biological products, which notice reflects approval by the agency of manufacture, use or sale for human administration.

### III. Examples

### Materials and Methods

### Reagents

GRP EIA, which is specific for the mature form of GRP (amide 1-27), was purchased from Phoenix Pharmaceuticals Inc. (Burlingame, CA, USA; catalog # EK-027-07). The GRP inhibitor, NSC77427, was made by the Small Molecule Library Reagent Program (National Cancer Institute, Division of Cancer Treatment and Diagnosis/Developmental Therapeutics Program, NIH). The GRPR antagonist, BW2258U89, was purchased from Phoenix Pharmaceuticals Inc. (Burlingame, CA, USA). The anti-GRP mouse monoclonal antibody, MoAb 2A11 (IgG1κ), was made by the National Cancer Institute, Center for Cancer Research, NCI-Navy Medical Branch and is also specific for the mature form of GRP [6]. Mouse IgG1κ isotype control antibody (MOPC21) was purchased from BioLegend (San Diego, CA, USA). Both the MOPC21 and control antibody preparations were confirmed to be endotoxin-free by a chromogenic Limulus Amoebocyte Assay (East Falmouth, MA, USA). The murine monoclonal mAb-2A11 (2A11) was provided by Dr. Cuttitta (NCI, NIH). Rabbit polyclonal anti-PGP 9.5 was obtained from Gene Technology Co., Ltd. (Shanghai, China).

### Mice and cotton rats

Six- to 8-week-old, WT C57BL/6J mice were purchased from the Jackson Laboratory (Bar Harbor, ME, USA). Four- to six-week-old, male or female cotton rats were obtained from the inbred colony maintained at Sigmovir Biosystems Inc. (SBI, Rockville, MD, USA). All animal experiments were conducted with institutional IACUC approvals from the University of Maryland, Baltimore and SBI.

### Viruses

Mouse-adapted H1N1 influenza A/PR/8/34 virus ("PR8") (ATCC, Manassas, VA, USA) was grown in the allantoic fluid of 10-day-old embryonated chicken eggs as described [47] and was kindly provided by Dr. Donna Farber (Columbia University). Non-adapted human influenza A/California/07/2009 strain (human pandemic H1N1) was kindly provided by Ted Ross (University of Pittsburgh). Preparation of human A/California 04/2009 (stock titer: 4.3 × 10⁷ TCID₅₀/ml) and A/Wuhan/359/95 (stock titer: 1 × 10⁷ TCID₅₀/ml) were previously described [24,48]. Human A/Victoria H3N2 (stock titer: 6.8 × 10⁶ TCID₅₀/ml) virus was obtained by harvesting the supernatants of Madin-Darby canine kidney (MDCK) cells inoculated 3 days previously at a low multiplicity of infection. Titers of all virus stocks were determined by standard endpoint dilution assays on MDCK cells as previously described [49].

### Virus challenge and treatment

For survival experiments, WT C57BL/6J mice were infected with mouse-adapted influenza virus, strain A/PR/8/34 (PR8; -7500 TCID₅₀, i.n., 25 µl/nares), a dose of PR8 that kills -90% of infected mice [16,17]. Two days after PR8 infection, mice received either vehicle (indicated in the figure legend), NSC77427 (20 µM; 100 µl intravenously (i.v.)), or BW2258U89 (20 µM; 100 µl i.v.) daily for five consecutive days (day 2 until day 6). For neutralizing antibody studies, mice received either IgGκ isotype control antibody (100 µg; 100 µl i.v.) or anti-GRP mouse MoAb 2A11 (IgG1κ; 100 µg; i.v.) on days 2 and 4 post infection. Mice were monitored daily for survival. In a separate assay, mice were infected with a non-adapted influenza strain, A/California/07/2009 H1N1 (∼10⁷ TCID₅₀, i.n., 25 µl/nares), a dose shown to kill -75% of infected mice [16]. Two days after PR8 infection, mice received either vehicle or NSC77427 (20 µM; 100 µl i.v.) daily for 5 consecutive days (day 2 until day 6). Mice were monitored daily for survival for 14 days. In some experiments, mice infected with PR8 were euthanized at days 2, 4, 6, or 8 post infection to harvest lungs for analysis of GRP protein levels by EIA, or for gene expression lung pathology, and IHC.

For cotton rat experiments, groups of five animals were infected as follows: with California pH1N1 and Wuhan H3N2 (1 × 10⁶ TCID₅₀), and with Victoria H3N2 (1 × 10⁵ TCID₅₀). Serum GRP levels were analyzed at 0, 4, 6, 8, 10, and 14 days post infection using the same GRP EIA Kit used for mouse studies.

### Histology and staining

Lungs were inflated and perfused and fixed with 4% paraformaldehyde. Fixed sections (5 µm) of paraffin-embedded lungs were stained with hematoxylin and eosin. Scoring of blinded slides by a board-certified pathologist was performed for severity of lung injury utilized multiple parameters, which were then added together: 0 = zero; 0.5 = rare (-1-2 cells per high power field (hpf)); 1 = few (∼3-4 cells/hpf); 2 = frequent (-5-10 cells/hpf); 3 = many (-11-20/hpf); 4 = over 20/hpf. For neutrophils (PMN), 5 = sheets of PMN associated with alveolar wall destruction. The cells evaluated were: marginating PMN in vasculature; PMN in alveolar spaces; mononuclear cells around conducting airways; and dead epithelial cells in conducting airways (mostly clusters).

### Immunohistochemistry

Immunohistochemical (IHC) staining for the mature GRP peptide (a PNEC granule marker) and PGP 9.5 (the neural/NE cytoplasmic isoform of ubiquitin-C-terminal hydrolase-1) was carried out as described previously [14]. In brief, formalin-fixed, paraffin-embedded lung sections were treated for 10 min with Triton X-100 (0.3% in phosphate-buffered saline (PBS)), then normal serum blocking. Diluted primary antibodies were added to sections overnight at 4 °C, followed by washing in PBS and incubation for 2 h at 4 °C with 1:200 dilution of biotinylated secondary antibodies (Vector, Burlingame, CA, USA). After blocking in 3% H₂O₂ in methanol, Vector ABC Elite was applied to slides for 30 min. Slides were developed by using diaminobenzidine and H₂O₂, and then counterstained in 2% aqueous methyl green. All slides were blinded for semi-quantitative analysis. Results are expressed as mean numbers of cells positive for PGP 9.5 or GRP per mm² lung tissue section. Statistical analysis was carried out using a one-tailed Student's *t* test.

### Quantitative real-time PCR

Total RNA isolation and quantitative real-time PCR (qRT-PCR) were performed as previously described [50]. Levels of mRNA for specific genes were normalized to the level of the housekeeping gene, *HPRT,* in the same samples and are expressed as "fold increase" over the relative gene expression measured in mock-infected lungs.

### Cytokine measurements

Cytokine levels were measured by enzyme-linked immunosorbent assay by the Cytokine Core Laboratory in the University of Maryland School of Medicine's Center for Innovative Biomedical Resources.

### Statistics

Statistical differences between two groups were determined using an unpaired, one-tailed Student's *t* test with significance set at *p* < 0.05. For comparisons between ≥3 groups, analysis was done by one-way analysis of variance followed by a Tukey's multiple comparison post hoc test with significance determined at *p* < 0.05. For survival studies, a log-rank (Mantel-Cox) test was used.

### Results

### Influenza infection induces GRP production in mice

WT C57BL/6J mice were infected intranasally (i.n.) with an ∼LD₉₀ of a mouse-adapted influenza strain, A/PR/8/34 (PR8) [16]. Mice were euthanized on days 2, 4, 6, and 8 post infection and the lungs harvested and homogenized for measurement of GRP levels by enzyme immunoassay (EIA). The level of GRP rose significantly in the lungs of PR8-infected mice starting 4 days post-infection and increased through day 8 post infection **(****Fig. 1a****).** Cotton rats (*Sigmodon hispidus*) have been used as an experimental model for human respiratory virus infections and are uniquely susceptible to non-adapted human strains of influenza [24]. GRP levels were measured in the sera of cotton rats in response to three influenza A strains, California pH1N1, Wuhan H3N2, and Victoria H3N2, at different times post-infection. All three strains induced significant increases in the levels of serum GRP over time before returning to basal levels by day 14 **(****Fig. 1b****).**

### Therapeutic administration of GRP inhibitors protect against PR8-induced lethality in mice

To determine if antagonism of GRP or the GRPR during PR8 infection would affect survival of mice, WT C57BL/6J mice were infected with PR8 (∼LD₉₀) on day 0. On day 2 post-infection, the GRP small-molecule inhibitor, NSC77427, was administered once daily for 5 consecutive days. Mice were monitored daily for survival. Mice treated with NSC77427 were significantly protected from PR8-induced lethality (53.3% survival, while only -7% of mice treated with vehicle survived after infection (p < 0.0002; **Fig. 2a**). Similar results were seen in mice infected with a non-adapted human strain, AlCalifornia/07/2009 H1N1, and treated with NSC77427 **(****Fig. 7****).** To confirm these findings, mice were treated with a highly specific anti-GRP monoclonal antibody (MoAb 2A11) [4] on days 2 and 4 post-PR8 infection. Anti-GRP IgG1κ (100 µg/mouse), but not an isotype control IgG1κ (MOPC21), protected mice against lethal infection (50% survival; p < 0.0001), confirming the protection observed with NSC77427 **(****Fig. 2b****).** In addition, the efficacy of treatment of influenza-infected mice with the small-molecule GRPR antagonist, BW2258U89, was similarly evaluated. Therapeutic administration of the GRPR antagonist once daily from days 2 to 6 post infection resulted in -60% survival compared to treatment with vehicle alone (10%) (p < 0.033; **Fig. 2c**). Taken together, these data strongly support a role for GRP in the lethal response to influenza infection.

### GRP antagonism ameliorates lung inflammatory response to influenza infection

Therapeutic treatment of PR8-infected mice with the small molecule GRP inhibitor, NSC77427, also led to a reduction in influenza-induced lung pathology **(****Fig. 3****),** in the absence of a significant change in log₁₀ viral titers (6.84 ± 0.1 tissue culture infectious dose 50% (TCID₅₀) in PR8-infected, vehicle-treated mice vs. 6.01 ± 0.4 TCID₅₀ in PR8-infected, NSC77427-treated mice). Briefly, groups of mice were infected with PR8 and subsequently treated with vehicle or NSC77427 from days 2 to 6 post-infection as described for **Fig. 2a****.** Mice were euthanized on day 6, 3 h after the final treatment. Mock-infected, vehicle-treated mice had normal lung architecture with intact airway epithelium, clear bronchi/bronchioles, and alveoli **(****Fig. 3a****).** Mice infected with influenza PR8 developed two distinct populations of inflammatory infiltrates: moderate collections of mononuclear cells surrounding conducting airways, mainly large-sized and medium-sized bronchioles, and dense nests of neutrophils in colony-like structures throughout the alveoli **(****Fig. 3b****).** At higher magnification **(****Fig. 3c****),** neutrophils are seen invading the alveoli in a vasculo-centric distribution, often in continuity with the pleural surface. PR8-infected mice that were treated with NSC77427 had either no inflammation, minimal collections of mononuclear cells surrounding conducting airways, or scattered neutrophils without the formation of nests and without evidence of alveolar destruction **(****Fig. 3d****).** The mean lung injury score based on blinded histopathology, as described in the Materials and methods section, was significantly different between mice infected and treated with vehicle only vs. mice infected then treated with NSC77427 **(****Fig. 3e****;** p = 0.002).

In the lung, PNECs are the classical cell type that produces GRP [25]. GRP is not visualized in normal PNECs in the mouse in the absence of oxidative stress or inflammation [25]. To assess whether PNEC numbers increased following PR8 infection, immunohistochemical (IHC) staining techniques were used that targeted two select neural/neuroendocrine marker antigens consisting of protein gene product 9.5 (PGP 9.5, cytoplasmic marker for ubiquitin hydrolase) and fully mature amidated GRP **(****Fig. 4****).** In naive or vehicle-injected, uninfected mice, infrequent PGP 9.5+ PNECs, GRP- cells are observed in conducting airways, usually bronchioles **(****Fig. 4a****),** consistent with observations in other laboratories [25]. PGP 9.5+ intrapulmonary nerves are also visible (asterisk in **Fig. 4a**)**,** but all pulmonary nerves are GRP- **(****Fig. 4b****).** At 6 days post-PR8 infection **(****Fig. 4c****),** there is ~6-8-fold increase in the number of stainable PGP+ PNECs (image analysis in **Fig. 4g****),** together with an ~6-fold increase in the appearance of GRP+ PNECs **(****Fig. 4d****;** image analysis in **Fig. 4g**), both primarily in the form of linear hyperplasia in small bronchioles. In PR8-infected mice, there is also extracellular GRP immunostaining consistent with GRP secretion. Mice infected with PR8, then treated with NSC77427 **(****Fig. 4e**, **f**), had -50% fewer PGP+ PNECs (p = 0.009), a -70% decrease in GRP+ PNECs (p = 0.001) (image analysis in **Fig. 4g**) and visibly less GRP immunostaining in the extracellular matrix **(****Fig. 4f****).** These observations suggest that GRP may contribute to PNEC differentiation and/or its own synthesis, consistent with observations in fetal baboon lung [11]. Taken together, these observations indicate that antagonism of GRP function during PR8 infection results in a decrease in both the total number of PNECs and GRP production by the cells.

### Effect of GRP antagonism on TLR4 signaling in macrophages

It was previously shown that PR8 infection of mice elicits a strong inflammatory response characterized by a significant upregulation of cytokine and chemokine gene expression [16]. The lungs of PR8-infected mice treated with the NSC77427 also showed blunted mRNA expression for genes that encode the pro-inflammatory cytokines interleukin-1β (IL-1β) and tumor necrosis factor-α (TNF-α), as well as interferon regulatory factor-3 (IRF-3)-dependent genes that encode interferon-β (IFN-β), and the chemokine, regulated upon activation normal T cell expressed and secreted (RANTES) **(****Fig. 5****).** Similar results were observed at the level of cytokine protein levels in lung homogenates from PR8-infected, NSC77427-treated mice **(****Fig. 8****).**

Petronilho et al. [22] previously showed that a different GRPR inhibitor than used in the present study, RC-3095, reduced TLR4 mRNA expression, blunted TLR4 signaling, and reduced production of IL-6 and monocyte chemoattractant protein-1 (MCP-1) in the RAW 264.7 murine macrophage cell line stimulated with the prototype TLR4 agonist, LPS. In addition, they found that RC-3095, when administered immediately after surgery, inhibited cytokine/chemokine production in rats in a model of polymicrobial sepsis induced by cecal ligation and puncture (CLP). These authors correlated serum GRP levels with disease severity in septic patients. Since it was previously reported that TLR4-/- mice are refractory to PR8 infection [15,16], and it was shown that multiple TLR4 antagonists protect WT mice from lethal PR8 infection [16-19], it was initially sought to determine if the GRP inhibitor, NSC77427, would exert an inhibitory effect on TLR4 signaling. WT mouse primary macrophages were pretreated with the GRP inhibitor, NSC77427, for 1 h (0.5 µM, a dose of NSC77427 that blocked GRP-induced angiogenesis *in vitro* and *in vivo*) [7], followed by LPS stimulation for 2 h. NSC77427 did not block LPS-induced MyD88-dependent (IL-1β, TNF-α) or TRIF-dependent (IFN-β, RANTES) gene expression **(****Fig. 9A****).** Likewise, treatment of macrophages with the GRPR antagonist, BW2258U89 (1 µM), for 1 h prior to LPS stimulation had no effect on LPS signaling **(****Fig. 9B****).** These data suggest either that the effect of GRP is not directly on macrophages or that macrophages are not the primary source of GRP, as supported by the IHC staining data presented herein **(****Fig. 4g****).** Since macrophages have been shown to express the GRPR [26], the hypothesis that exogenous GRP would modulate TLR4-dependent cytokine gene expression was tested. To this end, WT macrophages were treated with low doses of LPS (0.1 and 1 ng/ml, respectively) in the absence or presence of increasing doses of GRP (1-100 nM) for 2 h and gene expression measured. While these concentrations of GRP alone again did not induce cytokine gene expression, addition of GRP to suboptimal doses of LPS resulted in synergistic induction of both MyD88-dependent (IL-1β and TNF-α) and TRIF-dependent (MyD88-independent; IFN-β and RANTES) cytokine gene expression **(****Fig. 6****).** This synergistic effect was largely lost at higher doses of LPS **(****Fig. 10****).**

### Effect of GRP Antagonism on Macrophage Activity

In additional studies, the activity of GRP antagonism of macrophage activity, based on certain macrophage markers were performed. mRNA levels for CD68 (a macrophage marker), TLR4, and CCR2 (a marker of infiltrating monocytes) in RNA prepared from lungs of mice that were untreated (mock), infected but not treated (NT), or infected and treated with the GRP inhibitor NSC77427 were measured. Mice were mock infected or infected with influenza A/PR/8/34 (PR8) and then treated with saline (NT) or NSC77427 once daily from day 2 to day 6 post-infection. The results are shown in **Figure 11****.** Each point represents an individual mouse.

In addition, immunohistochemistry (IHC) for the detection of CD68 and TLR4 was carried out on the lung sections from the same mice. The results are provided in **Figure 12** and the numbers on the y-axis represent the mean ± SEM of the number of cells per 20X field that were stained with anti-CD69 or anti-TLR4 antibody. **p* = 0.0117; ***p* = 0.0069.

### REFERENCES

All patents and publications mentioned in this specification are indicative of the level of skill of those skilled in the art to which the invention pertains. All of the following references have been cited in this application:
1. Jensen, R. T., Battey, J. F., Spindel, E. R. & Benya, R. V. International union of pharmacology. LXVIII. Mammalian bombesin receptors: nomenclature, distribution, pharmacology, signaling, and functions in normal and disease states. Pharmacol. Rev. 60, 1-41 (2008).
2. Ramos-Alvarez, I. et al. Insights into bombesin receptors and ligands: highlighting recent advances. Peptides 72, 128-144 (2015).
3. Jaeger, N., Czepielewski, R. S., Bagatini, M., Porto, B. N. & Bonorino, C. Neuropeptide gastrin-releasing peptide induces PI3K/reactive oxygen species-dependent migration in lung adenocarcinoma cells. Tumor Biol. 39, 1-11 (2017).
4. Cuttitta et al. Bombesin-like peptides can function as autocrine growth factors in human small-cell lung cancer. Nature 316, 823-826 (1985).
5. Moody et al. BW2258U89: a GRP receptor antagonist which inhibits small cell lung cancer growth. Life Sci. 56, 521-529 (1995).
6. Martinez, A. et al. Identification of vasoactive nonpeptide positive and negative modulators of adrenomedulin using a neutralizing antibody-based screening strategy. Endocrinology 145, 3858-3865 (2004).
7. Martinez, A., Zudaire, E., Julián, M., Moody, T. W. & Cuttitta, F. Gastrin-releasing peptide (GRP) induces angiogenesis and the specific GRP blocker 77427 inhibits tumor growth in vitro and in vivo. Oncogene 24, 4106-4113 (2005).
8. Sunday, M. E., Yoder, B. A., Cuttitta, F., Haley, K. J. & Emanuel, R. L. Bombesin-like peptide mediate lung injury in a baboon model of bronchopulmonary dysplasia. J. Clin. Invest. 102, 584-594 (1998).
9. Gosney, J. R., Sissons, M. C., Allibone, R. O. & Blakey, A. F. Pulmonary endocrine cells in chronic bronchitis and emphysema. J. Pathol. 157, 127-133 (1989).
10. Meloni, F. et al. Bombesin enhances monocyte and macrophage activities: possible role in the modulation of local pulmonary defenses in chronic bronchitis. Respiration 63, 28-34 (1996).
11. Sunday, M. W. Oxygen, gastrin-releasing peptide, and pediatric lung disease: life in the balance. Front. Pediatr. 72, https://doi.org/10.3389/fped.2014.00072 (2014).
12. Moody, T. W., Pert, C. B., Gazdar, A. F., Carney, D. N. & Minna, J. D. High levels of intracellular bombesin charactize human small-cell lung carcinoma. Science 214, 1246-1248 (1981).
13. Subramaniam, M. et al. Bombesin-like peptides modulate alveolarization and angiogenesis in bronchopulmonary dysplasia. Am. J. Respir. Crit. Care Med. 176, 902-912 (2007).
14. Zhou, S. et al. Radiation-induced lung injury is mitigated by blockade of gastrin-releasing peptide. Am. J. Pathol. 182, 1248-1254 (2013).
15. Nhu, Q. M. et al. Novel signaling interactions between proteinase-activated receptor 2 and Toll-like receptors in vitro and in vivo. Mucosal Immunol. 3, 29-39 (2010).
16. Shirey, K. A. et al. The TLR4 antagonist Eritoran protects mice from lethal influenza infection. Nature 497, 498-502 (2013).
17. Shirey, K. A. et al. Novel strategies for targeting innate immune responses to influenza. Mucosal Immunol. 9, 1173-1182 (2016).
18. Piao, W. et al. A decoy peptide that disrupts TIRAP recruitment to TLRs is protective in a murine model of influenza. Cell. Rep. 11, 1941-1952 (2015).
19. Perrin-Cocon, L. et al. TLR4 antagonist FP7 inhibits LPS-induced cytokine production and glycolytic reprogramming in dendritic cells, and protects mice from lethal influenza infection. Sci. Rep. 7, https://doi.org/10.1038/srep40791 (2017).
20. Li, A., Potts-Kant, E. N., Garantziotis, S., Foster, W. M. & Hollingsworth, J. W. Hyaluronon signaling during ozone-induced injury requires TLR4, MyD88, and TIRAP. PLoS ONE 6, https://doi.org/10.1371/journal.pone.0027137 (2011).
21. Mathews, J. A. et al. Augmented responses to ozone in obese mice require IL-17A and gastrin-releasing peptide. Am. J. Respir. Cell. Mol. Biol. 58, 341-351 (2018).
22. Petronilho, F. et al. Gastrin-releasing peptide receptor antagonism induces protection from lethal sepsis: involvement of toll-like receptor 4 signaling. Mol. Med. 18, 1209-1219 (2012).
23. Abdulkhalek, S., Guo, M., Amith, S. R., Jayanth, P. & Szewczuk, M. R. G-protein coupled receptor antagonists mediate Neu1 sialidase and matrix metalloproteinase-9 cross-talk to induce transactivation of TOL-like receptors and cellular signaling. Cell Signal. 24, 2035-2042 (2012).
24. Blanco, J. C. et al. Receptor characterization and susceptibility of cotton rats to avian and 2009 pandemic influenza virus strains. J. Virol. 87, 2036-2045 (2013).
25. Polak, J. M. et al. Lung endocrine cell markers, peptides, and amines. Anat. Rec. 236, 169-171 (1993).
26. Czepieleski, R. S. et al. Gastrin-releasing peptide receptor (GRPR) mediates chemotaxis in neutrophils. Proc. Natl. Acad. Sci. USA 109, 547-552 (2012).
27. Cutz, E., Chan, W., Wong, V. & Conen, P. E. Ultrastructure and fluorescence histochemistry of endocrine (APUD-type) cells in tracheal mucosa of human and various animal species. Cell Tissue Res. 158, 425-437 (1975).
28. McDonald, T. et al. A gastrin releasing peptide from the porcine nonantral gastric tissue. Gut 19, 767-774 (1978).
29. Schnabel, E., Mains, R. E. & Farquhar, M. G. Proteolytic processing of pro-ACTH/endorphin begins in the Golgi complex of pituitary corticotropes and AtT-20 cells. Mol. Endocrinol. 3, 1223-1235 (1989).
30. Blalock, J. E., Harbour-McMenamin, D. & Smith, E. M. Peptide hormones shared by the neuroendocrine and immunologic systems. J. Immunol. 135, 858s-861s (1985).
31. Kiess, W. & Belohradsky, B. H. Endocrine regulation of the immune system. Klin. Wochenschr. 64, 1-7 (1986).
32. Zudaire, E. et al. Adrenomedullin is a cross-talk molecule that regulates tumor and mast cell function during human carcinogenesis. Am. J. Pathol. 168, 280-291 (2006).
33. Erbelding, E. J. et al. A universal influenza vaccine: the strategic plan for the national institute of allergy and infectious diseases. J. Infect. Dis. 218, 347-354 (2018).
34. Rajão, D. S. & Pérez, D. R. Universal vaccines and vaccine platforms to protect against influenza viruses in humans and agriculture. Front. Microbiol. 9, https://doi.org/10.3389/fmicb.2018.00123 (2018).
35. Gubareva, L. V. et al. Global update on the susceptibility of human influenza viruses to neuraminidase inhibitors, 2015-2016. Antivir. Res. 146, 12-20 (2017).
36. Centers for Disease Control and Prevention (CDC). Update: influenza activity-United States, October 1, 2017-February 3, 2018. Morb. Mortal. Wkly. Rep. 67, 169-179 (2018).
37. Centers for Disease Control and Prevention (CDC). Antiviral agents for the treatment and chemoprohylaxis of influenza. Morb. Mortal. Recomm. Rep. 60, 1-26 (2011).
38. Yang, H. et al. MD-2 is required for disulfide HMGB1-dependent TLR4 signaling. J. Exp. Med. 212, 5-14 (2015).
39. Kong, Y. et al. Functional diversity of notch family genes in fetal lung development. Am. J. Lung Cell. Mol. Physiol. 286, L1075-L1083 (2004).
40. Li, C. et al. Apc deficiency alters pulmonary epithelial cell fate and inhibits Nkx2.1 via triggering TGF-beta signaling. Dev. Biol. 378, 13-24 (2013).
41. Emanual, R. L. et al. Bombesin-like peptides and receptors in normal fetal baboon lung: roles in lung growth and maturation. Am. J. Physiol. 227, L1003-L1017 (1999).
42. Shapira, S. D. et al. A physical and regulatory map of host-influenza interactions reveals pathways in H1N1 infection. Cell 139, 1255-1267 (2009).
43. Aguayo, S. M. Determinants of susceptibility to cigarette smoke. Potential roles for neuroendocrine cells and neuropeptides in airway inflammation, airway wall remodeling, and chronic airflow obstruction. Am. J. Crit. Care Med. 149, 1692-1698 (1994).
44. Pardo, A. et al. Increase of lung neutrophils in hypersensitivity pneumonitis is associated with lung fibrosis. Am. J. Respir. Crit. Care Med. 161, 1698-1704 (2000).
45. Segura-Valdez, L. et al. Upregulation of gelatinases A and B, collagenases 1 and 2, and increased parenchymal cell death in COPD. Chest 117, 684-694 (2000).
46. Corbel, M., Biochot, E. & Lagente, V. Role of gelatinases MMP-2 and MMP-9 in tissue remodeling following acute lung injury. Braz. J. Med. Biol. Res. 33, 749-754 (2000).
47. Teijaro, J. R. et al. Costimulation modulation uncouples protection from immunopathology in memory T cell response to influenza virus. J. Immunol. 182, 6834-6843 (2009).
48. Ottolini, M. G. et al. The cotton rat provides a useful small-animal model for the study of influenza virus pathogenesis. J. Gen. Virol. 86, 2823-2830 (2005).
49. Patel, M. C., Shirey, K. A., Boukhvalova, M. S., Vogel, S. N. & Blanco, J. C. G. Serum high-mobility-group box 1 as a biomarker and a therapeutic target during respiratory virus infections. mBio 9, https://doi.org/10.1128/mBio.00246-18 (2018).
50. Shirey, K. A., Cole, L. E., Keegan, A. D. & Vogel, S. N. Francisella tularensis live vaccine strain induces macrophage alternative activation as a survival mechanism. J. Immunol. 181, 4159-4167 (2008).
51. Martinez, A., Julián, M., Bregonzio, C., Notari, L., Moody, T.W., & Cuttitta, F. Identification of vasoactive nonpeptidic positive and negative modulators of adrenomedullin using a neutralizing antibody-based screening strategy. Endocrinology 145(8), 3858-3865 (2004).

## Claims

1. A GRP inhibitor or a GRPR antagonist, or a combination thereof, for use in
i) reducing a virally-induced inflammatory response in a subject infected with a virus;
ii) reducing virally-induced lung damage in a subject infected with a virus;
iii) inhibiting virally-induced lethality in a subject infected with a virus and at risk for virally-induced lethality;
iv) treating viral infection in a subject infected with a virus; or
v) treating viral pneumonia in a subject having viral pneumonia,
wherein the subject is infected with an influenza virus, wherein the influenza virus is an Influenza A virus, an Influenza B virus, an Influenza C virus or an Influenza D virus,
wherein the GRP inhibitor is one or more of the mouse anti-GRP neutralizing monoclonal antibody 2A11 (MoAb 2A11) and the small-molecule inhibitors NSC77427, NSC54671, NSC112200, and 2,5-dibromo-3,6-dimethylcyclohexa-2,5-diene-1,4-dione, and
wherein the GRPR antagonist is the water-soluble peptide BW2258U89.

2. The GRP inhibitor or GRPR antagonist or combination thereof for the use of claim 1, wherein the inflammatory response is pulmonary inflammation.

3. The GRP inhibitor or GRPR antagonist or combination thereof for the use of claim 1, wherein said treating viral infection or viral pneumonia is to reduce one or more of pulmonary inflammation, lung damage, decreased PNECs, GRP gene expression, GRP protein production, GRPR activation, GRPR signaling, inflammatory cytokine and/or chemokine production, and HMGB1 release in the subject in comparison to a subject infected with the virus but not receiving treatment.

4. The GRP inhibitor or GRPR antagonist or combination thereof for the use of any one of claims 1 to 3, wherein
i) the Influenza A virus is serotype H1N1, California pH1N1 strain, H1N2, H2N2, H2N3, H3N1, H3N2, Wuhan H3N2 strain, Victoria H3N2 strain, H3N8, H5N1, H5N2, H5N3, H5N6, H5N8, H5N9, H6N1, H6N2, H7N1, H7N2, H7N3, H7N4, H7N7, H7N9, H9N2, or H10N7; or
ii) the Influenza B virus is serotype Victoria or Yamagata.

## Patentansprüche

1. GRP-Inhibitor oder GRPR-Antagonist, oder eine Kombination davon, zur Verwendung bei
i) Reduzieren einer viral induzierten Entzündungsreaktion bei einem mit einem Virus infizierten Subjekt;
ii) Reduzieren viral induzierter Lungenschäden bei einem mit einem Virus infizierten Subjekt;
iii) Hemmen viral induzierter Letalität bei einem Subjekt, das mit einem Virus infiziert ist und bei dem das Risiko einer viral induzierten Letalität besteht;
iv) Behandeln einer Virusinfektion bei einem mit einem Virus infizierten Subjekt; oder
v) Behandeln einer Viruspneumonie bei einem Subjekt das Viruspneumonie aufweist,
wobei das Subjekt mit einem Influenzavirus infiziert ist, wobei das Influenzavirus ein Influenza-A-Virus, ein Influenza-B-Virus, ein Influenza-C-Virus oder ein Influenza-D-Virus ist,
wobei der GRP-Inhibitor einer oder mehrere der monoklonalen Anti-GRP-Neutralisierungs-Antikörper 2A11 (MoAb 2A11) der Maus und die niedermolekularen Inhibitoren NSC77427, NSC54671, NSC112200 und 2,5-Dibrom-3,6-dimethylcyclohexa-2,5-dien-1,4-dion ist, und
wobei der GRPR-Antagonist das wasserlösliche Peptid BW2258U89 ist.

2. GRP-Inhibitor oder GRPR-Antagonist oder Kombination davon zur Verwendung nach Anspruch 1, wobei die Entzündungsreaktion Lungenentzündung ist.

3. GRP-Inhibitor oder GRPR-Antagonist oder Kombination davon zur Verwendung nach Anspruch 1, wobei das Behandeln der viralen Infektion oder Viruspneumonie Reduzieren einer oder mehrerer von Lungenentzündung, Lungenschädigung, verringerten PNECs, GRP-Genexpression, GRP-Proteinproduktion, GRPR-Aktivierung, GRPR-Signalisierung, entzündlicher Zytokin- und/oder Chemokinproduktion und HMGB1-Freisetzung in dem Subjekt im Vergleich zu einem Subjekt, das mit dem Virus infiziert ist, aber keine Behandlung erhält, ist.

4. GRP-Inhibitor oder GRPR-Antagonist oder Kombination davon zur Verwendung nach einem der Ansprüche 1 bis 3, wobei
i) das Influenza-A-Virus vom Serotyp H1N1, Kalifornien pH1N1 Stamm, H1N2, H2N2, H2N3, H3N1, H3N2, Wuhan H3N2 Stamm, Victoria H3N2-Stamm, H3N8, H5N1, H5N2, H5N3, H5N6, H5N8, H5N9, H6N1, H6N2, H7N1, H7N2, H7N3, H7N4, H7N7, H7N9, H9N2, oder H10N7 ist; oder
ii) das Influenza-B-Virus vom Serotyp Victoria oder Yamagata ist.

## Revendications

1. Inhibiteur de GRP ou antagoniste de GRPR, ou combinaison de ceux-ci, à utiliser pour
i) réduire une réponse inflammatoire d'origine virale chez un sujet infecté par un virus ;
ii) réduire les lésions pulmonaires d'origine virale chez un sujet infecté par un virus ;
iii) inhiber la létalité d'origine virale chez un sujet infecté par un virus et présentant un risque de létalité d'origine virale ;
iv) traiter une infection virale chez un sujet infecté par un virus ; ou
v) traiter une pneumonie virale chez un sujet atteint de pneumonie virale,
dans lequel le sujet est infecté par un virus de la grippe, dans lequel le virus de la grippe est un virus de la grippe A, un virus de la grippe B, un virus de la grippe C ou un virus de la grippe D,
dans lequel l'inhibiteur de GRP est un ou plusieurs parmi l'anticorps monoclonal neutralisant anti-GRP de souris 2A11 (MoAb 2A11) et les inhibiteurs à petites molécules NSC77427, NSC54671, NSC112200 et 2,5-dibromo-3,6-diméthylcyclohexa-2,5-diène-1,4-dione, et
dans lequel l'antagoniste de GRPR est le peptide hydrosoluble BW2258U89.

2. Inhibiteur de GRP ou antagoniste de GRPR ou combinaison de ceux-ci à utiliser selon la revendication 1, dans lequel la réponse inflammatoire est une inflammation pulmonaire.

3. Inhibiteur de GRP ou antagoniste de GRPR ou combinaison de ceux-ci à utiliser selon la revendication 1, dans lequel ledit traitement d'une infection virale ou d'une pneumonie virale consiste à réduire une ou plusieurs parmi une inflammation pulmonaire, des lésions pulmonaires, des PNEC moindres, une expression de gène GRP, une production de protéine GRP, une activation de GRPR, une signalisation de GRPR, une production de cytokine et/ou de chimiokine inflammatoire et une libération de HMGB1 chez le sujet par comparaison à un sujet infecté par le virus mais ne recevant pas de traitement.

4. Inhibiteur de GRP ou antagoniste de GRPR ou combinaison de ceux-ci à utiliser selon l'une quelconque des revendications 1 à 3, dans lequel
i) le virus de la grippe A est de sérotype H1N1, souche California pH1N1, H1N2, H2N2, H2N3, H3N1, H3N2, souche Wuhan H3N2, souche Victoria H3N2, H3N8, H5N1, H5N2, H5N3, H5N6, H5N8, H5N9, H6N1, H6N2, H7N1, H7N2, H7N3, H7N4, H7N7, H7N9, H9N2 ou H10N7 ; ou
ii) le virus de la grippe B est de sérotype Victoria ou Yamagata.
